Europäisches Patentamt

European Patent Office

Office européen des brevets

(19)

(11) Publication number: **0 212 090**
**B1**

(12) EUROPEAN PATENT SPECIFICATION

(45) Date of publication of patent specification: **09.01.91**

(51) Int. Cl.⁵: **A 61 B 8/06**

(21) Application number: **86107241.1**

(22) Date of filing: **28.05.86**

(54) Doppler blood velocimeter with range continuity.

(30) Priority: **30.05.85 US 739254**

(43) Date of publication of application:
**04.03.87 Bulletin 87/10**

(45) Publication of the grant of the patent:
**09.01.91 Bulletin 91/02**

(84) Designated Contracting States:
**AT BE CH DE FR GB IT LI LU NL SE**

(56) References cited:
**EP-A-0 137 317**
**WO-A-85/02105**
**DE-A-2 641 498**
**US-A-4 217 909**
**US-A-4 417 584**

(73) Proprietor: **Advanced Technology Laboratories, Inc.**
**22100 Bothell Highway, S.E.**
**Bothell Washington 98041-3003 (US)**

(72) Inventor: **DesJardins, Philip A.**
**8201-20th Avenue, N.E.**
**Seattle Washington (US)**
Inventor: **Powers, Jeffry E.**
**3639 Pleasant Beach Drive**
**Bainbridge Island Washington (US)**

(74) Representative: **Vossius & Partner**
**Siebertstrasse 4 P.O. Box 86 07 67**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to an improved medical ultrasound Doppler unit of the type used for measuring velocity of blood flow.

In particular, the invention relates to a pulsed Doppler ultrasound velocimeter and to an anti-aliasing method for use with multigate Doppler means.

Pulsed Doppler velocimeters can discern only a limited range of Doppler-shifted frequencies. This limitation arises from insufficient time-sampling of the Doppler signal. Pulsed Doppler velocimeters sample a Doppler-shifted signal at a single arbitrary depth, a depth determined by the delay between the insonifying pulse and the sampling time. This sampling, performed at a rate called the Pulsed Repetition Frequency (PRF) limits the maximum unambiguously discernable Doppler-shifted frequency, and, therefore, the maximum discernable velocity.

The Nyquist Sampling Theorem implies that a pulsed Doppler velocimeter can unambiguously discern only those Doppler-shifted frequencies which are between −PRF/2 and +PRF/2. Any Doppler-shifted frequency outside of this interval (hereafter called the "Nyquist interval") will be aliased, that is, it will appear to be at a frequency that is inside this interval. Without additional information, the pulsed Doppler velocimeter cannot discern whether a perceived Doppler-shifted frequency is actually within the Nyquist interval or whether it is an alias of a frequency outside of this interval.

Mathematically, the perceived frequency, $f_p$, of a signal sampled at $f_s$ and having a true frequency, $f_t$, is found by:

$$f_p = f_t - f_s * \text{ROUND}(f_t/f_s) \qquad \text{(eq 1)}$$

where ROUND(X) is a function which rounds the number inside the parentheses, i.e., ROUND(X) will be an integer which is equal to the greatest integer in X, plus 1 if the remaining fractional part is greater than or equal to 0.5.

For pulsed Doppler velocimeters, this equation is written as:

$$f_p = f_t - \text{PRF} * \text{ROUND}(f_t/\text{PRF}) \qquad \text{(eq 2)}$$

Thus, the perceived frequency is always within the Nyquist interval. If the true frequency is also between −PRF/2 and PRF/2, then no aliasing occurs, and the perceived frequency is the true frequency. If the true frequency is outside the Nyquist interval, then the true frequency appears to be the perceived frequency as found in (eq. 2). The ROUND function in the above equation can alias several possible true frequencies ($f_t$'s) onto the same perceived frequency $f_p$. Accordingly, there is no direct way to determine whether the perceived frequency is the true frequency or one of many possible aliases.

Methods used heretofore to circumvent the aliasing problem included using continuous wave (CW) Doppler, a common technique. However, continuous wave Doppler loses all range resolution.

Another approach is to deccrease the transmitted frequency in order to proportionally decrease the Doppler-shifted frequency. A disadvantage of this common technique is the problem of decreased scattering and decreased spatial resolution.

Increasing the PRF in order to increase the Nyquist interval is also a commonly used technique. However, it is subject to range ambiguities. The signal from a desired range cell of depth, d, is sampled by delaying the sample with respect to the insonifying pulse by a time, t, found by:

$$t = 2d/c, \qquad \text{(eq 3)}$$

Where c is the propagation velocity of the insonifying signal. Equation 3 does not separate the desired range's signal from signals coming from deeper ranges (which were insonified by prior pulses). That is, it also receives signals from ranges at the following depths:

$$d(n) = (c/2) * (t + nT), \qquad \text{(eq 4)}$$

where T is the pulse repetition period (=1/PRF), and n is an integer which is greater than or equal to 1.

This interference is not a problem when T is sufficiently large (the PRF is sufficiently low). In such case, the unwanted range cells are so deep that their signals are sufficiently attenuated by the propagating medium and, therefore, are so weak that they do not interfere with the desired signal. However, as the PRF increases, the unwanted ranges move closer to the insonifying source, and their signals become strong enough to interfere with the desired signal.

This interference has been exploited to advantage by the high-PRF or "extended range" concept, whereby the desired range is not the closest received range but rather one of the deeper ones. This technique assumes, however, that the signals coming from the shallower, unwanted ranges are negligible.

The main disadvantage of the extended-range concept is that we do not known that the interference from undesired ranges is negligible.

WO—A1—85/02105 discloses a Doppler ultrasound apparatus for providing two-dimensional blood flow data. The probe includes an array of transducers that produce an ultrasound beam that can be electrically scanned over a sector. For each angle scanned, a burst of energy is transmitted and the energy reflected by the blood is periodically sampled by range gates. As a result, blood flow data at a plurality of points along a plurality of scan lines are produced. According to WO—A1—85/02105, the sampling frequency or pulse repetition rate should be at least twice the frequency of the maximum Doppler shift produced by the blood. As a result, aliasing will not occur and data will not be erroneously interpreted. By employing a high pulse repetition rate, however, the range of the system is limited because signals reflected by blood distant from the transducers are unable to make it back to the array before the next pulse is transmitted.

Another approach which was used heretofore involves estimating the Doppler frequency $f(i)$ at a time $t(i)$, and assuming that estimates at times $t(j)$, near $t(i)$, are also close to (within $\pm PRF/2$ of) this estimate. If a perceived frequency, $f_p(j)$, is outside this range, it is replaced with the "most-likely" true frequency, $f_{ml}(j)$. $f_{ml}(j)$ is chosen from all the possible true frequencies which alias to the perceived frequency $f_p(j)$. The one chosen is the one closest to $f(i)$ and is found by the following formula:

$$f_{ml}(j)=f_p(j)+PRF*ROUND((f_{ml}(i)-f_p(j))/PRF) \qquad \text{(eq 5)}$$

The baseline estimate, $f(i)$, is periodically updated in order to track non-stationary Doppler spectra (such as caused by the variation of blood velocity distribution over the cardiac cycle, as seen by Doppler blood velocimeters).

One disadvantage of this approach is that the original baseline estimate must not be aliased, or else the future estimates will be corrupted.

A second disadvantage of this method is that the technique assumes that the time between frequency estimates is sufficiently short such that the difference between the true frequencies $f_t(j)$ and $f_t(i)$ is within the Nyquist interval. If the Doppler spectrum changes sufficiently between updates, then future estimates will be corrupted.

It is an object of the invention to avoid the above disadvantages and to provide a Doppler ultrasound velocimeter and an anti-aliasing method which allow reliable measurements of the velocity of blood flow and anti-aliasing.

Range-continuity anti-aliasing corrects for aliasing by first making a frequency estimate, $f(i)$, at an arbitrary range cell $r(i)$. Any perceived frequency $f_p(j)$ estimated at a nearby range cell $r(j)$ is compared to $f(i)$ and corrected for aliasing according to equation 5. The most likely frequency, $f_{ml}(j)$, at range cell $r(j)$ is found by:

$$f_{ml}(j)=f_p(j)+PRF*ROUND((f_{ml}(i)-f_p(j))/PRF) \qquad \text{(eq 6)}$$

After $f_{ml}(j)$ is calculated, the frequency estimates for ranges close to $r(j)$ can be alias-corrected by extending the technique of equation 6. Thus, $r(j)$ and $f_{ml}(j)$ become the new $r(i)$ and $f(i)$, respectively, and a new range is chosen as $r(j)$.

By successively choosing the new range cells, the $r(j)$'s, further and further away from the original $f(i)$, all range cells acquired by the Doppler velocimeter can be corrected for aliasing.

This technique assumes that the original $f(i)$, called $f(0)$, is correct to within $\pm PRF/2$. This can be done by choosing a range $r(0)$ where it is assumed there are no moving targets. The frequency $f(0)$ estimated at this range is assumed to be within the Nyquist interval and is set to $f_p(0)$.

Similarly, if the Doppler velocimeter determines that the signal coming from an arbitrary range cell has no significant Doppler information, then the correction circuitry assumes that the signal is noise and sets the most-likely frequency to be the perceived frequency.

This technique requires the ability to simultaneously acquire Doppler-shifted signals from multiple range cells. Accordingly, the use of this technique requires a "multigate Doppler". This technique requires a frequency estimator that operates at any or all the acquired range cells. This frequency estimator calculates the perceived frequency $f_p$, a frequency which is within the Nyquist interval.

The disclosed technique assumes that the true Doppler-shifted frequency varies slowly in range. The distance between $r(i)$ and $r(j)$ must be sufficiently short such that the difference between the true frequencies, $f_t(i)$ and $f_t(j)$, is within $\pm PRF/2$. This means there must be sufficient spatial sampling in range.

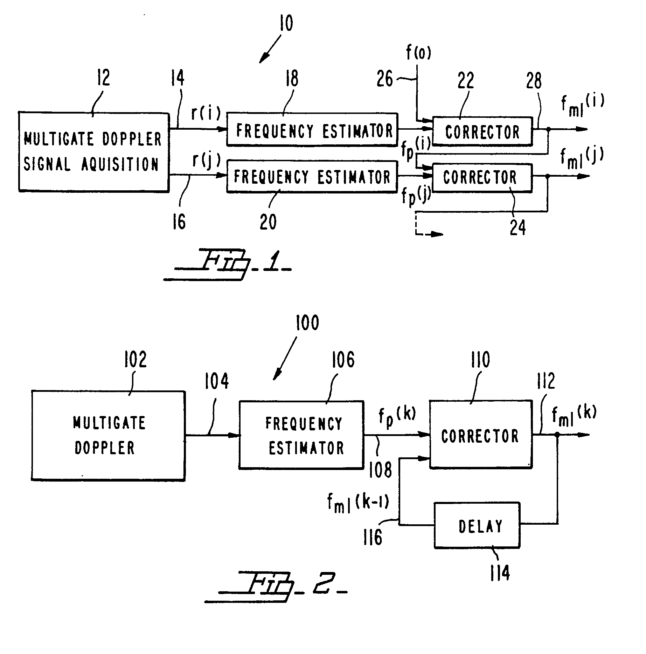

Referring generally to Fig. 1, a first embodiment of the present invention 10 is shown. The invention 10 is comprised of a multigate Doppler unit 12. As used herein, the term "multigate Doppler unit" refers to an apparatus which may be used to acquire Doppler signals at multiple depth ranges. Devices of this type have been used heretofore and are considered, for the purposes of this invention, to be well known to those of ordinary skill in the art. The outputs of the multigate Doppler unit 12 consist of Doppler signals at various depth ranges. Thus, an output signal at range i appears on a first line 14, and an output signal range j on a second line 16. These output siganls, are (i) and (j), respectively. These output signals go into frequency estimators 18, 20. A frequency estimator is a device capable of estimating frequency of the Doppler signal. The output of each of the frequency estimators 18, 20 is the perceived frequency $f_p(i)$ and $f_p(j)$, respectively

and will be in the range of ±PRF/2. As discussed above, the perceived frequency may be aliased. Accordingly, the perceived frequency outputs of the frequency estimators 18, 20 are fed into frequency corrector circuits 22, 24. The job of the frequency corrector circuits 22, 24 is to correct the perceived frequency, $f_p$, by adding the proper multiple of the PRF which is found by using equation 6.

In accordance with the present invention, the proper multiple will be an integer which is found iteratively. In order to arrive at the most likely frequency, the assumption is made that the velocity in the selected depth range will not vary significantly from the velocity of the preceding depth range. This means that if the output of corrector 22 is $f_{ml}(i)$ then the output of corrector 24 will be $f_p(j)$ plus a multiple of PRF found by rounding the difference between $f_{ml}(i)$ and $f_p(j)$ to the integer corresponding to the nearest multiple of PRF. In general, each output frequency will be correct if two assumptions are correct. First, the blood flow must be within the sample depth and has not changed significantly from the blood flow in the adjacent sample depth, i.e., the Doppler signal is within ±PRF/2 of the Doppler signal of the adjacent sample depth. Second, since each correction is based upon the accuracy of the preceding frequency, at some point we have to assume that we have properly initialized a corrector. Accordingly, the present invention assumes that there is no movement in the shallowest range and the initial most likely frequency, $f_{ml}(0)$ of the first corrector 22 is set to $f_p(0)$ on line 26. Thereafter, $f_{ml}(i)$ on line 28 is used as the correcting frequency which is input into the corrector 24 to compute $f_{ml}(j)$.

Any time that intensity of the Doppler signal is weaker than a predefined threshold, the corrector assumes that there is no blood flow at the corrector depth and $f_{ml}$ out of that corrector will be set to $f_p$.

Referring now to Fig. 2, the preferred embodiment 100 of the present invention is shown. In the preferred embodiment 100 there is a multigate Doppler unit 102 which generates time-multiplexed samples of the Doppler sent via output line 104 into a frequency estimator 106. For example, the multigate Doppler unit 102 may start with the most shallow depth and then step deeper into successive sample groups from adjacent sample depths to provdie a frequency estimator 106 with Doppler signals. The output of the frequency estimator 106 will be a perceived frequency $f_p(k)$ for each depth k. That perceived frequency $f_p(k)$ is set into a corrector circuit 110 via line 108, and output of the corrector circuit 110 will be the most likely frequency depth k, $f_{ml}(k)$ on line 112. $f_{ml}(k)$ is also set into a delay unit 114 which samples the $f_{ml}(k)$ and holds it while the multigate Doppler unit 102 steps into the next depth. Accordingly, the output of the delay unit 114 will be the most likely frequency at depth k-1 on line 116.

In accordance with the preferred embodiment of the invention 100, the assumption is made that at depth 0 there is no blood flow. Accordingly, the frequency $f_{ml}(0)$ is initialized to $f_p(0)$, and subsequent depths are adjusted to provide the most likely frequency thereafter.

## Claims

1. A pulsed Doppler ultrasound velocimeter comprising:
   (a) multigate Doppler means (12; 102) including means for transmitting and receiving ultrasonic waves and means for providing Doppler signals from a plurality of different range gates corresponding to a plurality of different sample depths; and
   (b) frequency estimator means (18, 20; 106) for receiving the Doppler signals and for providing, for each range gate, a corresponding perceived frequency signal representing the perceived frequency of the Doppler signal for the range gate; characterized by
   (c) anti-aliasing means (22, 24; 110, 114) for receiving the perceived frequency signals and for providing an unaliased signal corresponding to each range gate, the anti-aliasing means (22, 24; 110, 114) including means for comparing, for each range gate, the perceived frequency signal corresponding to the range gate with the unaliased signal corresponding to a different range gate, and for modifying the value of the perceived frequency signal corresponding to the range gate to produce the unaliased signal corresponding to the range gate by reducing the difference between the unaliased signal corresponding to said different range gate and the perceived frequency signal corresponding to the range gate, and initialization means for providing an initialization signal such that for one range gate, the anti-aliasing means (22,24; 110, 114) produces the corresponding unaliased signal by reducing the difference between the initailization signal and the perceived frequency signal corresponding to said one range gate.

2. The velocimeter of Claim 1, wherein the multigate Doppler means (12; 102) operates at a pulse repetition frequency PRF, and wherein the anti-aliasing means (22, 24; 110, 114) includes means for modifying the value of the perceived frequency signal to produce the unaliased signal by adding to the value of the perceived frequency signal the product of the PRF and a positive, negative or zero integer.

3. The velocimeter of Claim 1, wherein the multigate Doppler means (102) provides the Doppler signals as a time multiplexed series wherein successive Doppler signals correspond to adjacent range gates, wherein the frequency estimator means (106) provides the perceived frequency signals as a corresponding series of time multiplexed signals for adjacent range gates, and wherein the anti-aliasing means (110, 114) includes delay means (114) for storing the unaliased signal for each range gate for use in the succeeding production of the unaliased signal for the succeeding range gate.

4. The velocimeter of Claim 3, wherein the initialization means provides the initialization signal to the anti-aliasing means (110, 114) for the production of the unaliased signal for the first range gate.

5. An anti-aliasing method for use with multigate Doppler means that includes means for transmitting and receiving ultrasonic energy and means for providing Doppler signals from a plurality of different range gates corresponding to a plurality of different sample depths, the method comprising:

(a) providing, for each range gate, a corresponding perceived frequency signal representing the perceived frequency of the Doppler signal for the range gate;

(b) providing an unaliased signal corresponding to each range gate by comparing, for the range gate, the perceived frequency signal corresponding to the range gate with the unaliased signal corresponding to a different range gate, and modifying the value of the perceived frequency signal corresponding to the range gate to produce the unaliased signal corresponding to the range gate by reducing the difference between the unaliased signal corresponding to said different range gate and the perceived frequency signal corresponding to the rage gate; and

(c) providing an initialization range such that for one range gate, the anti-aliasing means produces the corresponding unaliased signal by reducing the difference between the initialization signal and the perceived frequency signal corresponding to said one range gate.

6. The method of Claim 5, wherein the multigate Doppler means operates at a pulse repetition frequency PRF, and wherein the value of the perceived frequency signal is modified to produce the unaliased signal by adding to the value of the perceived frequency signal the product of the PRF and a positive, negative or zero integer.

7. The method of Claim 5, wherein the Doppler signals are provided as a time multiplexed series wherein successive Doppler signals correspond to adjacent range gates, wherein the perceived frequency signals are provided as a corresponding series of time multiplexed signals for adjacent range gates, and wherein the unaliased signal for each range gate is stored for use in the succeeding production of the unaliased signal for the succeeding range gate.

8. The method of Claim 7, wherein the initialization signal is provided to the anti-aliasing means for the production of the unaliased signal for the first range gate.

## Patentansprüche

1. Gepulster Doppler-Ultraschall-Geschwindigkeitsmesser mit:

(a) einer Mehrfach-Gate-Dopplereinrichtung (12; 102) mit einer Einrichtung zum Übertragen und Empfangen von Ultraschallwellen und einer Einrichtung zum Liefern von Dopplersignalen von mehreren unterschiedlichen Bereichsgates, die mehreren unterschiedlichen Probentiefen entsprechen;

(b) einer Frequenzschätzeinrichtung (18, 20; 106) zum Empfangen der Dopplersignale und zum Liefern eines entsprechenden wahrgenommenen Frequenzsignals für jedes Bereichsgate, das die wahrgenommene Frequenz des Dopplersignals für das Bereichsgate repräsentiert; gekennzeichnet durch

(c) eine Anti-Aliasing-Einrichtung (22, 24; 110, 114) zum Empfangen der wahrgenommenen Frequenzsignale und zum Liefern eines jedem Bereichsgate entsprechenden unverfälschten Signals, wobei die Anti-Aliasing-Einrichtung (22, 24; 110, 114) eine Einrichtung zum Vergleichen des dem Bereichsgate entsprechenden wahrgenommenen Frequenzsignals mit dem einem unterschiedlichen Bereichsgate entsprechenden unverfälschten Signal für jedes Bereichsgate und zum Modifizieren des Werts des dem Bereichsgate entsprechenden wahrgenommenen Frequenzsignals aufweist, um das dem Bereichsgate entsprechende unverfälschte Sigcal durch Verringern der Differenz zwischen dem dem unterschiedlichen Bereichsgate entsprechenden unverfälschten Signal und dem dem Bereichsgate entsprechenden wahrgenommenen Frequenzsignal zu erzeugen, und eine Initialisierungseinrichtung zum Liefern eines Initialisierungssignals, so daß für eine Bereichsgate die Anti-Aliasing-Einrichtung (22, 24; 110, 114) das entsprechende unverfälschte Signal durch Verringern der Differenz zwischen dem Initialisierungssignal und dem diesem Bereichsgate entsprechenden wahrgenommenen Frequenzsignal erzeugt.

2. Geschwindigkeitsmesser nach Anspruch 1, wobei die Mehrfach-Gate-Dopplereinrichtung (12; 102) bei einer Impulsfolgefrequenz PRF arbeitet, wobei die Anti-Aliasing-Einrichtung (22, 24; 110, 114) eine Einrichtung zum Modifizieren des Werts des wahrgenommenen Frequenzsignals enthält, um das unverfälschte Signal durch Addieren des Produkts der Impulsfolgefrequenz PRF und einer positiven oder negativen ganzen Zahl oder Null zu dem Wert des wahrgenommenen Frequenzsignals zu erzeugen.

3. Geschwindigkeitsmesser nach Anspruch 1, wobei die Mehrfach-Gate-Dopplereinrichtung (102) die Dopplersignale als Zeitmultiplexreihe liefert, wobei aufeinanderfolgende Dopplersignale benachbarten Bereichsgates entsprechen, wobei die Frequenzschätzeinrichtung (106) die wahrgenommenen Frequenzsignale als entsprechende Reihe von Zeitmultiplexsignalen für benachbarte Bereichsgates liefert, und wobei die Anti-Aliasing-Einrichtung (110, 114) eine Verzögerungseinrichtung (114) zum Speichern des unverfälschten Signals für jedes Bereichsgate zur Verwendung in der nachfolgenden Erzeugung des unverfälschten Signals für den nachfolgenden Bereichsgate enthält.

4. Geschwindigkeitsmesser nach Anspruch 3, wobei die Initialisierungseinrichtung das Initialisierungssignal der Anti-Aliasing-Einrichtung (110, 114) zum Erzeugen des unverfälschten Signals für das erste Bereichsgate zuführt.

5. Anti-Aliasing-Verfahren zur Verwendung mit einer Mehrfach-Gate-Dopplereinrichtung, die eine Einrichtung zum Übertragen und Empfangen von Ultraschallenergie und eine Einrichtung zum Liefern von Dopplersignalen von mehreren unterschiedlichen Bereichsgates enthält, die mehreren unterschiedlichen Probentiefen entsprechen, wobei das Verfahren die folgenden Schritte aufweist:

5

(a) Liefern eines entsprechenden wahrgenommenen Frequenzsignals für jedes Bereichsgate, das die wahrgenommene Frequenz des Dopplersignals für das Bereichsgate repräsentiert;

(b) Liefern eines jedem Bereichsgate entsprechenden unverfälschten Signals durch Vergleichen, für jedes Bereichsgate, des dem Bereichsgate entsprechenden wahrgenommenen Frequenzsignals mit dem einem unterschiedlichen Bereichsgate entsprechenden, unverfälschten Signal und Modifizieren des Werts des dem Bereichsgate entsprechenden wahrgenommenen Frequenzsignals zum Erzeugen des dem Bereichsgate entsprechenden unverfälschten Signals durch Verringern der Differenz zwischen dem dem unterschiedlichen Bereichsgate entsprechenden unverfälschten Signal und dem dem Bereichsgate entsprechenden wahrgenommenen Frequenzsignal; und

(c) Liefern eines Initialisierungssignals, so daß für ein Bereichsgate die Anti-Aliasing-Einrichtung das entsprechende unverfälschte Signal durch Verringern der Differenz zwischen dem Initialisierungssignal und dem diesem Bereichsgate entsprechenden wahrgenommenen Frequenzsignal erzeugt.

6. Verfahren nach Anspruch 5, wobei die Mehrfach-Gate-Dopplereinrichtung bei einer Impulsfolgefrequenz PRF arbeitet, und wobei der Wert des wahrgenommenen Frequenzsignals modifiziert wird, um das unverfälschte Signal durch Addieren des Produkts der Impulsfolgefrequenz PRF und einer positiven oder negativen ganzen Zahl oder Null zu dem Wert des empfangenen Frequenzsignals zu erzeugen.

7. Verfahren nach Anspruch 5, wobei die Dopplersignale als Zeitmultiplexreihe geliefert werden, wobei aufeinanderfolgende Dopplersignale benachbarten Bereichsgates entsprechen, wobei die wahrgenommenen Frequenzsignale als entsprechende Reihe von Zeitmultiplexsignalen für benachbarte Bereichsgates geliefert werden, und wobei das unverfälschte Signal für jedes Bereichsgate zur Verwendung in der nachfolgenden Erzeugung des unverfälschten Signals für das nachfolgende Bereichsgate gespeichert wird.

8 Verfahren nach Anspruch 7, wobei das Initialisierungssignal zum Erzeugen des unverfälschten Signals für das erste Bereichsgate der Anti-Aliasing-Einrichtung zugeführt wird.

**Revendications**

1. Un appareil Doppler de mesure de vitesse par ultra-sons pulsés comprenant:

(a) des moyens Doppler à portes multiples (12, 102) comprenant des moyens pour émettre et recevoir des ondes ultrasoniques et des moyens pour envoyer des signaux Doppler à partir de plusieurs portes de plages différentes correspondant à plusieurs profondeurs d'échantillons différentes, et

(b) des moyens d'estimation de fréquence (18, 20, 106) pour recevoir les signaux Doppler et fournir, pour chaque porte de plage, un signal de fréquence correspondant perçu représentant la fréquence perçue du signal Doppler pour la porte de fréquence; caractérisé par

(c) des moyens antirepliement (anti-alia sing) (22, 24, 110, 114) pour recevoir les signaux de fréquence perçus et pour fournir un signal non replié (unaliased) correspondant à chaque porte de plage, les moyens antirepliement (22, 24, 110, 114) comprenant des moyens pour comparer, pour chaque porte de plage, le signal de fréquence perçu correspondant à la porte de plage avec le signal non replié correspondant à une porte de plage différente, et pour modifier la valeur du signal de fréquence perçu correspondant à la porte de plage pour produire le signal non replié correspondant à la porte de plage en réduisant la différence entre le signal non replié correspondant à ladite différente porte de plage et le signal de fréquence perçu correspondant à la porte de plage, et des moyens d'initialisation pour envoyer un signal d'initialisation tel que, pour une porte de plage, les moyens antirepliement (22, 24, 110, 114) produisent le signal non replié correspondant en réduisant la différence entre le signal d'initialisation et le signal de fréquence perçu correspondant à ladite porte de plage.

2. Un appareil de mesure de vitesse selon la revendication 1, dans lequel les moyens Doppler à portes multiples (12, 102) fonctionne à une fréquence de répétition d'impulsions PRF, et dans lequel les moyens antirepliement (22, 24, 110, 114) comprennent des moyens pour modifier la valeur du signal de fréquence perçu pour produire le signal non replié en ajoutant à la valeur du signal de fréquence perçu le produit de PRF et d'un entier positif, négatif ou nul.

3. Un appareil de mesure de vitesse selon la revendication 1, dans lequel le moyen Doppler à portes multiples (102) fournit les signaux Doppler sous la forme d'une série multiplexée dans le temps dans laquelle des signaux Doppler successifs correspondent à des portes de plages adjacentes, dans lequel le moyen d'estimation de fréquence (106) fournit les signaux de fréquence perçus sous forme d'une série correspondante de signaux multiplexés dans le temps pour des portes de plages adjacentes, et dans lequel le moyen antirepliement (110, 114) comprend un moyen de retard (114) pour mémoriser, pour chaque porte de plage, le signal non replié à utiliser dans la production suivante du signal non replié pour la porte de plage suivante.

4. Un appareil de mesure de vitesse selon la revendication 3 dans lequel le moyen d'initialisation fournit le signal d'initialisation aux moyens antirepliement (110, 114) pour la production du signal non replié pour la première porte de plage.

5. Un procédé antirepliement à utiliser avec un moyen Doppler à portes multiples et qui comprend des moyens pour transmettre et recevoir une énergie ultrasonique et des moyens pour envoyer des signaux Doppler à partir de plusieurs portes de plages différentes, correspondant à plusieurs profondeurs d'échantillon, le procédé comprenant les étapes consistant à:

(a) fournir, pour chaque porte de plage, un signal de fréquence perçu correspondant représentant la fréquence perçue du signal Doppler pour la porte de plage;

(b) fournir un signal non replié correspondant à chaque porte de plage en comparant, pour la porte de plage, le signal de fréquence perçu correspondant à la porte de plage avec le signal non replié, correspondant à une porte de plage différente, et modifier la valeur du signal de fréquence perçu correspondant à la porte de plage pour produire le signal non replié correspondant à la porte de plage en réduisant la différence entre le signal non replié correspondant à ladite porte de plage différente et le signal de fréquence perçu correspondant à la porte de plage; et

(c) fournir un signal d'initialisation tel que, pour une porte de plage, le moyen antirepliement produit le signal correspondant non replié en réduisant la différence entre le signal d'initialisation et le signal perçu de fréquence correspondant à ladite porte de plage.

6. Un procédé selon la revendication 5, dans lequel le moyen Doppler à portes multiples fonctionne à une fréquence de répétition d'impulsions PRF, et dans lequel la valeur du signal de fréquence perçu est modifiée pour produire le signal non replié en ajoutant à la valeur du signal de fréquence perçu le produit du PRF et d'un entier positif, négatif ou nul.

7. Un procédé selon la revendication 5, dans lequel les signaux Doppler sont fournis sous forme de séries multiplexées dans le temps dans lesquelles des signaux Doppler successifs correspondent à des portes de plages adjacentes, dans lequel les signaux de fréquence perçus sont fournis sous la forme d'une série correspondante de signaux multiplexés dans le temps pour des portes de plages adjacentes, et dans lequel le signal non replié pour chaque porte de plage est mémorisé pour être utilisé dans une production ultérieure du signal non replié pour la porte de plage suivante.

8. Un procédé selon la revendication 7, dans lequel le signal d'initialisation est fourni aux moyens antirepliement pour la production du signal non replié pour la première porte de plage.

_Fig._1_

_Fig._2_

1